(19) **Europäisches Patentamt**
**European Patent Office**
**Office européen des brevets**

(11) **EP 1 891 329 B1**

(12) **FASCICULE DE BREVET EUROPEEN**

(45) Date de publication et mention
de la délivrance du brevet:
**30.03.2011 Bulletin 2011/13**

(51) Int Cl.:
**F04B 43/04** *(2006.01)*     **B01F 13/00** *(2006.01)*

(21) Numéro de dépôt: **06778944.6**

(86) Numéro de dépôt international:
**PCT/FR2006/050566**

(22) Date de dépôt: **16.06.2006**

(87) Numéro de publication internationale:
**WO 2006/134307 (21.12.2006 Gazette 2006/51)**

(54) **DISPOSITIF DE POMPAGE PAR ELECTROMOUILLAGE ET APPLICATION AUX MESURES D'ACTIVITE ELECTRIQUE**

ELEKTROBENETZUNGS-PUMPVORRICHTUNG UND IHRE VERWENDUNG ZUM MESSEN ELEKTRISCHER AKTIVITÄT

ELECTROWETTING PUMPING DEVICE AND USE FOR MEASURING ELECTRICAL ACTIVITY

(84) Etats contractants désignés:
**AT BE BG CH CY CZ DE DK EE ES FI FR GB GR HU IE IS IT LI LT LU LV MC NL PL PT RO SE SI SK TR**

(30) Priorité: **17.06.2005 FR 0551662**

(43) Date de publication de la demande:
**27.02.2008 Bulletin 2008/09**

(73) Titulaire: **Commissariat à l'Énergie Atomique et aux Énergies Alternatives 75015 Paris (FR)**

(72) Inventeurs:
• **SAUTER-STARACE, Fabien**
  **F-38170 Seyssinet-Pariset (FR)**
• **BERTHIER, Jean**
  **F-38240 Meylan (FR)**

(74) Mandataire: **Poulin, Gérard et al BREVALEX 95 rue d'Amsterdam 75378 Paris Cedex 8 (FR)**

(56) Documents cités:
**EP-A- 1 386 657      WO-A-02/07503
WO-A-2004/029608    US-B2- 6 773 566**

EP 1 891 329 B1

**Description**

DOMAINE TECHNIQUE ET ART ANTÉRIEUR

L'invention concerne d'abord le domaine des techniques de pompage et de déplacement de micro volumes de liquide, tels que des micro-gouttes.

Le déplacement de fluides dans les systèmes micro-fluidiques requiert l'usage de dispositifs de pompage pouvant reposer sur différents principes physiques.

A cette échelle, les forces capillaires sont bien supérieures à la gravité. Plusieurs principes physiques ont été mis au point pour déplacer des fluides ou les mettre en pression.

Deux familles de principes se distinguent : ceux utilisant des parties mobiles pour mettre en mouvement les fluides par pression hydrostatique ou interaction fluide -structure (pompe péristaltique) et ceux n'utilisant pas de parties mobiles mais des forces physiques directement appliquées aux fluides

Parmi les principes s'inscrivant dans cette deuxième classe on peut citer : l'électro-osmose et la thermocapillarité.

L'électro-osmose requiert des tensions très élevées pour atteindre des pressions de plusieurs bars. Les champs électriques sont de l'ordre de 200 à 1000 V/cm. Des échauffements par effet Joule sont inhérents à ce champ électrique, d'où une contrainte de régulation thermique voire une incompatibilité avec les conditions de survie de matériels chimique ou biologique fragile.

Cet échauffement serait particulièrement critique dans le cas des mesures de résistivité de type « patch-clamp planaire ». Les conductivités des solutions salines sont de l'ordre de 0,5 à 4 S/m. Par ailleurs, des champs électriques importants (ordre de grandeur 200 à 8000 V/cm pour des cellules de poumon de souris) généreraient de l'électroporation, soit une perméabilité de la membrane cellulaire.

La présente invention se rapporte également à un procédé et un dispositif de mesure de l'activité électrique d'une ou plusieurs cellules biologiques et notamment à un dispositif de mesure de type « patch clamp ».

Pour étudier les activités électriques des cellules, la technique du « patch-clamp » a été proposée par Sakmann et Neher en 1981. Mais, récemment, des alternatives ont été recherchées pour augmenter le taux de réussite de cette mesure et augmenter le nombre de données accessibles.

Le document WO04/038409 décrit un dispositif pour réaliser de telles mesures. Ce dispositif est de type planaire, en silicium.

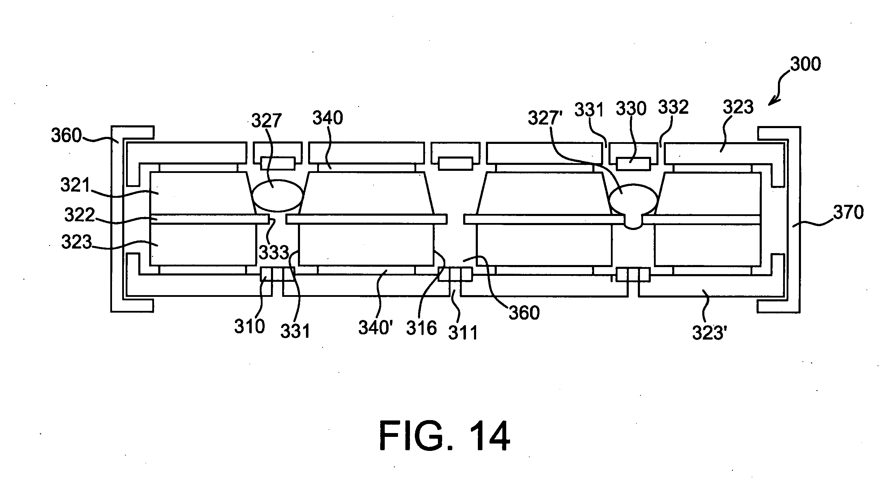

La figure 14 illustre un dispositif 300 de mesure de type « Patch-clamp » planaire tel que décrit dans le document WO 2004/038409A2.

Deux circuits imprimés 323, 323' assurent le confinement des fluides dans des chambres 326, 326' réalisées dans des plaques 321, 323, par exemple en silicium. Ces chambres sont remplies de solution d'électrophysiologie. Ces deux circuits sont munis d'électrodes 310, 330. Des joints 340, 340' assurent l'étanchéité du système.

Une couche intermédiaire 322 comporte un orifice 333 assurant une communication entre les chambres supérieure 326 et inférieure 326' et permettant la capture d'une cellule 327 par succion. L'ouverture 331 de la couche 323 est d'une taille supérieure à l'ouverture 333.

La puce réalisée selon ce document met en outre en oeuvre un système de conduits 311, 331, 332 permettant l'aspiration de fluides. Plus précisément ce dispositif comporte des canaux destinés à être connectés à des capillaires eux-mêmes connectés à des moyens d'aspiration de liquide situés en dehors de la puce. Le système est donc complexe, non compact.

Par ailleurs, les volumes aspirés sont difficilement contrôlables, et sont importants, de l'ordre de quelques dizaines de nanolitres à plusieurs microlitres.

Des mâchoires 360, 370 permettent de maintenir l'ensemble du système.

Dans ce type de dispositif, les volumes de fluide sont conditionnés par les cavités 326, 326', et par les joints réalisant l'étanchéité des chambres inférieures et supérieures. Il faut donc remplir individuellement, séquentiellement ou parallèlement, chaque site de mesure d'une solution adaptée à la mesure d'activité électrique des canaux ioniques et comportant une suspension cellulaire. Le volume de fluide est, là encore, important et la miniaturisation limitée par les standards des équipements de dispense. Cette contrainte limite aussi les possibilités d'intégration car chaque site doit être accessible à des moyens de dispense macroscopique.

Dans ce type de dispositif la gestion des dépressions nécessaires à la capture d'une cellule unique et à l'invagination de sa membrane plasmique ou cellulaire est donc obtenue grâce à un système macroscopique comprenant deux générateurs de pression permettant l'obtention d'une différence de pression contrôlée entre les chambres supérieures et inférieures.

Ce contrôle des fluides peut aussi être obtenu par des pompes ou des pousses seringues.

Ces systèmes sont macroscopiques et leur degré de parallélisation reste faible. Ils ne sont pas, non plus, à la fois parallélisables et adressables de façon indépendante pour chaque site.

Ces systèmes sont macroscopiques et ne sont pas compatibles avec l'augmentation du nombre de sites de mesure pourtant permise par les procédés de lithographie et de gravure collectif des micro-technologies.

Il se pose donc d'abord le problème de trouver un nouveau dispositif de pompage micro fluidique, en particulier qui puisse être compatible avec un dispositif de type « patch clamp » planaire.

Il se pose également le problème de trouver un nouveau dispositif de pompage micro fluidique ne présentant pas une ou plusieurs des limitations exposées ci-dessus.

Le document WO 2004/029608 décrit un dispositif pour déplacer des gouttelettes par électromouillage tel qu'il est défini par le préambule de la revendication 13.

## EXPOSÉ DE L'INVENTION

L'invention concerne un procédé de pompage, à travers un orifice d'un premier substrat, d'un premier volume de liquide, en contact avec une première surface hydrophobe dudit substrat, dans lequel une variation de pression entre le premier volume de liquide et un deuxième volume de liquide, situé en contact avec ledit orifice et une deuxième surface hydrophobe dudit substrat, est obtenue par électromouillage.

Le premier et/ou le deuxième volume de liquide peut être confiné, au moins lors du pompage, entre ladite première surface hydrophobe et/ou ladite deuxième surface hydrophobe et un deuxième et/ou un troisième substrat.

Un tel procédé peut mettre en oeuvre un dispositif d'électromouillage, comportant ledit premier substrat dont la première surface est hydrophobe ou recouverte d'une couche hydrophobe, et une pluralité d'électrodes disposées sous ladite couche hydrophobe, le pompage étant obtenu par activation de ces électrodes.

La deuxième surface du dispositif d'électromouillage peut comporter une couche hydrophobe, et une pluralité d'électrodes disposées sous ladite couche hydrophobe.

Les première et deuxième surfaces du substrat peuvent être parallèles entre elles et à un plan défini par le substrat, ou bien la première surface du substrat peut être parallèle à un plan défini par le substrat, la deuxième surface étant alors définie par au moins une portion de la paroi dudit orifice.

Le premier et/ou le deuxième volume de liquide peuvent être constitués d'une goutte de liquide, la ou les gouttes étant par exemple formés à partir d'un ou de plusieurs réservoirs, et pouvant avoir par exemple un volume compris entre 1 nl et 10 $\mu$l.

L'invention concerne un procédé d'analyse d'un liquide d'un premier volume de liquide comportant :

- la mise en contact de ce premier volume de liquide avec une surface hydrophobe,
- le pompage de ce premier volume de liquide à l'aide d'un deuxième volume de liquide, selon un procédé tel que décrit ci-dessus, afin de le positionner contre ledit orifice,
- une mesure d'activité électrique dudit liquide.

La mesure d'activité électrique peut être réalisée sur une cellule unique contenue dans le premier volume de liquide, la mesure étant par exemple une mesure sur un canal ionique de cellule ou un ou des canaux d'une membrane cellulaire.

Elle peut aussi être réalisée sur un objet biologique tel qu'un embryon ou un ovocyte de bovin.

L'invention concerne également un dispositif de pompage de volumes de liquide, comportant :

- un substrat, présentant une première et une deuxième surface, dont au moins une est hydrophobe, ou recouverte d'une couche hydrophobe, et au moins un orifice traversant ledit substrat,
- des moyens pour déplacer, par électromouillage, au moins un volume de liquide sur une desdites faces du substrat.

Ledit orifice peut comporter une première partie, ayant une première dimension maximale, et une deuxième partie, ayant une deuxième dimension maximale, supérieure à la première.

Les moyens de déplacement de gouttes par électromouillage peuvent comporter une pluralité d'électrodes disposées sous ladite surface ou couche hydrophobe.

La deuxième surface du dispositif d'électromouillage peut être hydrophobe ou comporter une couche hydrophobe, une pluralité d'électrodes pouvant être disposées sous ladite surface ou couche hydrophobe.

Les première et deuxième surfaces du substrat peuvent être parallèles entre elles et à un plan défini par le substrat, ou encore la première surface du substrat peut être parallèle à un plan défini par le substrat, la deuxième surface étant définie par au moins une portion de la paroi dudit orifice.

Un dispositif selon l'invention peut comporter en outre une première couche diélectrique, en partie sous la première surface ou couche hydrophobe, une deuxième couche diélectrique pouvant en outre être disposée sur une surface parallèle à la première surface.

Un deuxième substrat et/ou un troisième substrat peuvent être disposés en regard de la première et/ou de la deuxième surface hydrophobe.

Le deuxième et/ou le troisième substrat peuvent comporter en outre une couche hydrophobe superficielle, ainsi qu'une contre-électrode.

L'invention concerne également un dispositif d'analyse d'un premier volume de liquide, comportant au moins un dispositif tel que ci-dessus, et des moyens de mesure d'activité électrique d'un volume de liquide associés à au moins un orifice.

Les moyens de mesure d'activité électrique peuvent être associés à au moins un orifice comportant des première et deuxième électrodes.

Un dispositif selon l'invention peut comporter en outre au moins un réservoir de liquide et des moyens, par exemple des moyens de déplacement de gouttes par électromouillage, pour amener des gouttes de liquide depuis ce réservoir ou depuis au moins un de ces réservoirs vers l'un des orifices.

BRÈVE DESCRIPTION DES DESSINS

- Les figures 1A - 1C représentent le principe de déplacement de gouttes, par électromouillage,
- la figure 2 représente une configuration fermée de dispositif de déplacement de gouttes,
- les figures 3A et 3B représentent une configuration mixte de dispositif de déplacement de gouttes,
- les figures 4 et 5A - 5B représentent un dispositif de déplacement de gouttes, dans lequel le capot supérieur est muni d'une électrode,
- les figures 6A - 7B représentent divers modes de réalisation d'un dispositif de pompage selon l'invention,
- la figure 8 représente une vue détaillée d'un autre dispositif de pompage selon l'invention,
- les figures 9 - 11 représentent divers modes de réalisation d'un dispositif de mesure de type « patch clamp », selon l'invention,
- la figure 12 représente une vue de dessus d'un autre dispositif selon l'invention, comportant plusieurs sites de mesure,
- les figures 13A - 13D représentent un puits ou un réservoir de liquide,
- la figure 14 représente un dispositif de type patch - clamp selon l'art antérieur ;

EXPOSÉ DÉTAILLÉ DE MODES DE RÉALISATION PARTICULIERS

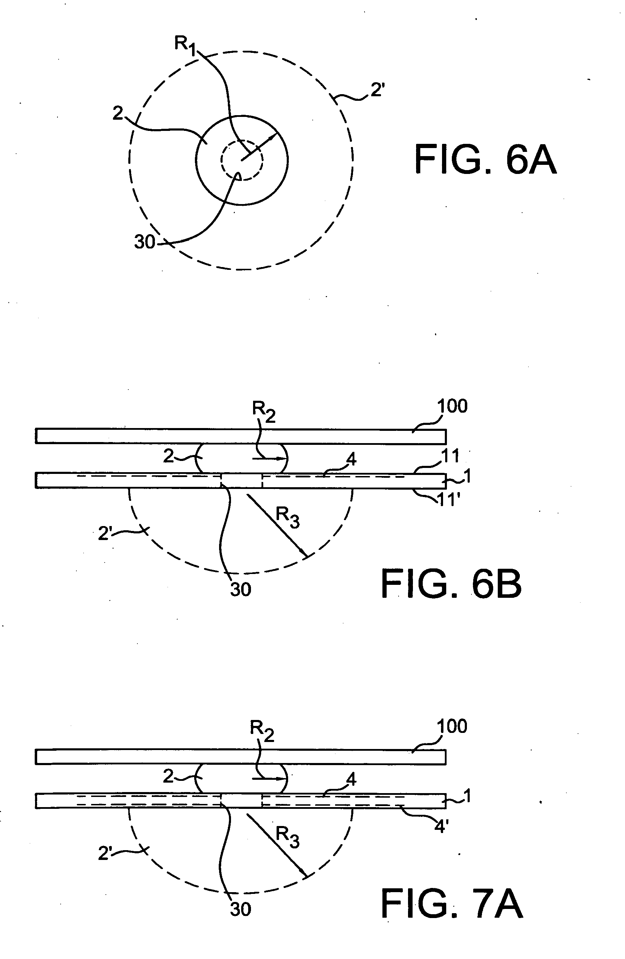

Un dispositif de pompage selon l'invention met en oeuvre un dispositif de déplacement ou de manipulation de gouttes de liquide, par électromouillage, comme décrit ci-dessous en liaison avec les figures 6A - 7B. Ces gouttes 2, 2' sont en contact avec une première et une deuxième face 11, 11' d'un substrat 1, muni d'une ouverture ou d'un orifice 30.

Le dispositif de pompage obtenu est donc compact et individualisé pour chaque site de mesure, permettant le pompage de faibles volumes de liquide en un site de pompage et ne nécessitant pas de moyens tels que des conduits d'aspiration de fluide.

Un tel dispositif peut en outre permettre la formation et l'acheminement des gouttes 2, 2' de liquide vers un site de pompage.

Dans un procédé et un dispositif de pompage selon l'invention, l'une des gouttes est amenée, par électromouillage, à avoir un volume plus important.

L'électromouillage permet de modifier l'angle de mouillage et le rayon de courbure de cette goutte ou des deux gouttes.

L'électromouillage permet également de modifier les pressions électrostatiques dans les gouttes. En effet, si, par électromouillage, on diminue l'angle de mouillage d'une des gouttes sur la surface de laquelle elle repose, le rayon de courbure de l'interface entre cette goutte et son environnement extérieur va augmenter, entraînant une diminution de la pression en son sein.

Réciproquement, si l'on augmente l'angle de mouillage de l'une des gouttes avec la surface avec laquelle elle est en contact, le rayon de courbure de l'interface entre cette goutte et son environnement extérieur va diminuer, entraînant une augmentation de la pression en son sein. La goutte se rétracte, et la pression y augmente, du fait de l'augmentation du rayon de courbure.

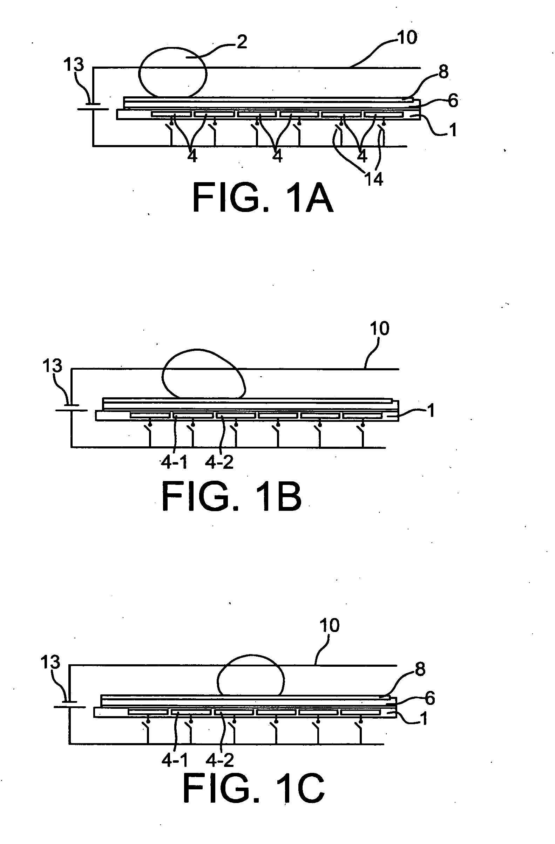

Un premier mode de réalisation d'un dispositif de déplacement et de manipulation de gouttes, de type système ouvert et qui peut être mis en oeuvre dans le cadre de l'invention, est illustré sur les figures 1A - 1C.

Ce mode de réalisation met en oeuvre un dispositif de déplacement ou de manipulation de gouttes de liquide reposant sur le principe de l'électromouillage sur un diélectrique.

Des exemples de tels dispositifs sont décrits dans l'article de M.G. Pollack, A.D. Shendorov, R.B. Fair, intitulé « Electro-wetting-based actuation of droplets for integrated microfluidics », Lab on Chip 2 (1) (2002) 96-101.

Les forces utilisées pour le déplacement de gouttes de liquide sont alors des forces électrostatiques.

Le document FR 2 841 063 décrit un dispositif mettant en oeuvre, en outre, un caténaire en regard des électrodes activées pour le déplacement.

Le principe de ce type de déplacement est synthétisé sur les figures 1A - 1C.

Une goutte 2 repose sur un réseau 4 d'électrodes, dont elle est isolée par une couche diélectrique 6 et une couche hydrophobe 8 (figure 1A).

Le caractère hydrophobe de cette couche signifie que la goutte a un angle de contact ou de mouillage, sur cette couche, supérieur à 90°.

Les électrodes 4 sont elles-mêmes formées en surface d'un substrat 1.

Lorsque l'électrode 4-1 située à proximité de la goutte 2 est activée, à l'aide de moyens 14 de commutation, dont la fermeture établit un contact entre cette électrode et une source de tension 13 via un conducteur commun 16, la couche diélectrique 6 et la couche hydrophobe 8 entre cette électrode activée et la goutte sous tension agissent comme une capacité.

La contre-électrode 10 permet un éventuel déplacement par électromouillage à la surface de la surface hydrophobe; elle maintient un contact électrique avec la goutte pendant un tel déplacement. Cette contre-électrode peut être soit un caténaire comme dans FR - 2 841 063, soit un fil enterré soit une électrode planaire dans le capot d'un système confiné (un tel système confiné est décrit plus loin).

En système ouvert, s'il n'y a pas de déplacement, il est possible d'étaler la goutte sur la surface hydrophobe, sans contre-électrode. C'est par exemple le cas si la goutte peut être amenée sur la surface hydrophobe par un système de dispense classique, les électrodes 4-1, 4-2 servant uniquement à étaler ou déformer la goutte à l'endroit où elle a été déposée.

La goutte peut ainsi être éventuellement déplacée de proche en proche (figure 1C), sur la surface hydrophobe 8, par activation successive des électrodes 4-1, 4-2,... etc, le long du caténaire 10.

Il est donc possible de déplacer des liquides, mais aussi de les mélanger (en faisant s'approcher des gouttes de liquides différents), et de réaliser des protocoles complexes.

Les documents cités ci-dessus donnent des exemples de mises en oeuvre de séries d'électrodes adjacentes pour la manipulation d'une goutte dans un plan, les électrodes pouvant en effet être disposées de manière linéaire, mais aussi en deux dimensions, définissant ainsi un plan de déplacement des gouttes.

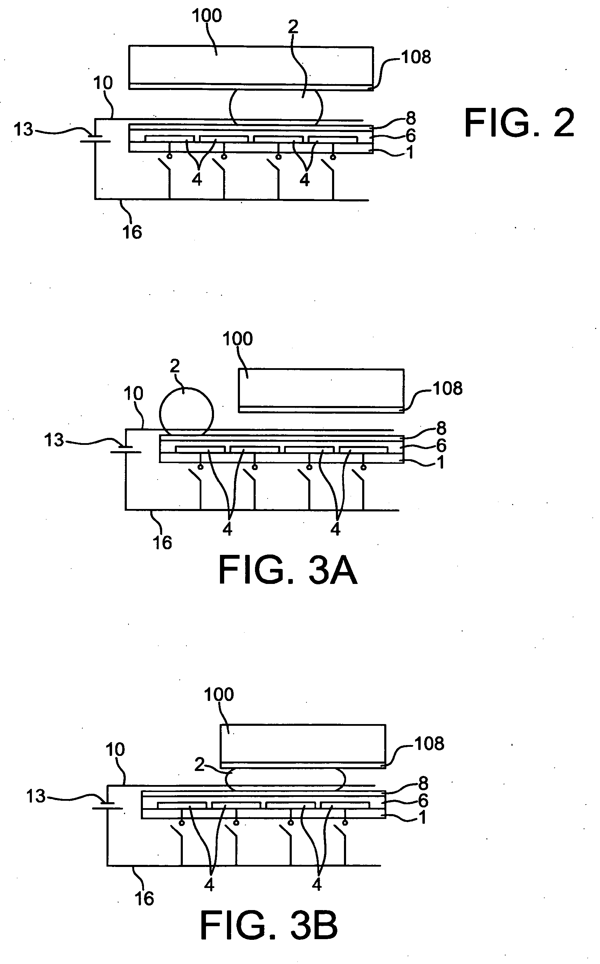

La figure 2 représente un autre mode de réalisation d'un dispositif de déplacement ou de manipulation de gouttes, de type système fermé ou confiné, pouvant être mis en oeuvre dans le cadre de l'invention.

Sur cette figure, des références numériques identiques à celles des figures 1A - 1C y désignent des mêmes éléments.

Ce dispositif comporte en outre un substrat supérieur 100, de préférence également recouvert d'une couche hydrophobe 108. Cet ensemble peut être éventuellement transparent, permettant une observation par le haut.

Les figures 3A et 3B, sur lesquelles des références numériques identiques à celles de la figure 2 y désignent des éléments identiques ou similaires, représentent un système mixte déplacement ou de manipulation de gouttes, dans lequel une goutte 2 est initialement en milieu ouvert (figure 3A), l'activation d'électrodes 4-1, 4-2, 4-3 permettant un aplatissement de la goutte (figure 3B), en système fermé, dans une zone où le système est muni d'un capot, comme illustré ci-dessus en liaison avec la figure 2.

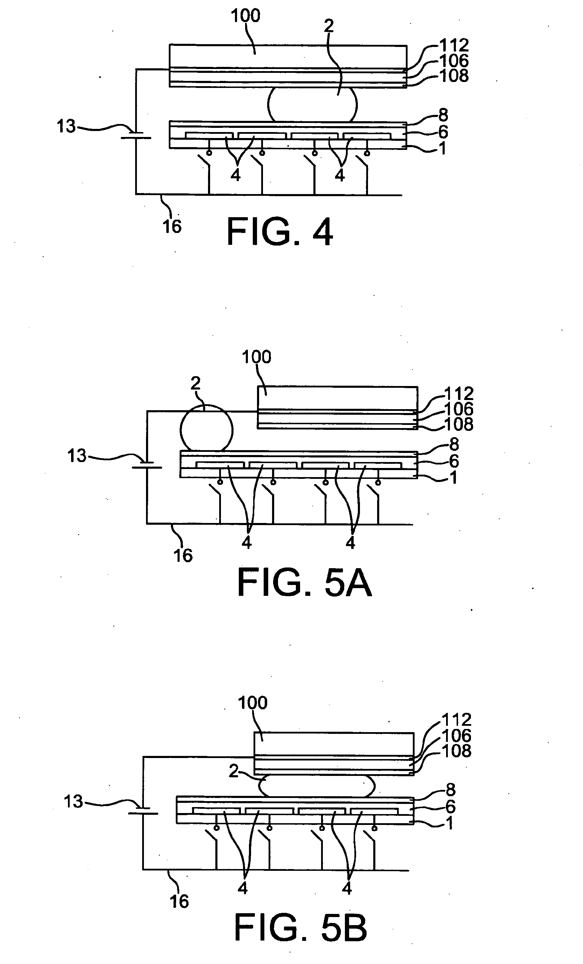

La figure 4 représente une variante du système fermé, avec un capot conducteur 100, comportant une électrode ou un réseau d'électrodes 112, ainsi qu'une couche isolante 106 et une couche hydrophobe 108.

Le caténaire 10 des figures précédentes est remplacé, dans ce mode de réalisation, par l'électrode 112. L'activation de cette électrode 112 et des électrodes 4 permet de déplacer la goutte dans la position voulue puis de l'étirer ou de la déformer, pour l'amener sur le trajet d'un faisceau lumineux 50.

Les figures 5A et 5B, sur lesquelles des références numériques identiques à celles de la figure 4 y désignent des éléments identiques ou similaires, représentent un système mixte, dans lequel une goutte 2 est initialement en milieu ouvert (figure 5A), l'activation d'électrodes 4-1, 4-2, 4-3 permettant un aplatissement de la goutte (figure 5B), en système fermé, dans une zone où le système est muni d'un capot, comme illustré ci-dessus en liaison avec la figure 4.

Un dispositif selon l'invention peut en outre comporter des moyens qui vont permettre de commander ou d'activer les électrodes 4, par exemple un ordinateur type PC et un système de relais connectés au dispositif ou à la puce, tels les relais 14 de la figure 1A, ces relais étant pilotés par les moyens de type PC.

Typiquement, la distance entre un éventuel conducteur 10 (figures 1A - 5B) d'une part et la surface hydrophobe 8 d'autre part est par exemple comprise entre 1 $\mu$m et 100 $\mu$m ou 500 $\mu$m.

Ce conducteur 10 peut se présenter par exemple sous la forme d'un fil de diamètre compris entre 10 $\mu$m et quelques centaines de $\mu$m, par exemple 200 $\mu$m. Ce fil peut être un fil d'or ou d'aluminium ou de tungstène ou d'autres matériaux conducteurs.

Lorsque deux substrats 1, 100 sont utilisés (figures 2 - 5B), ils sont distants d'une distance comprise entre, par exemple, 10 $\mu$m et 100 $\mu$m ou 500 $\mu$m.

Quel que soit le mode de réalisation considéré, une goutte de liquide 2 pourra avoir un volume compris entre, par exemple, 1 picolitre et quelques microlitres, par exemple entre 1 pl et 5 $\mu$l ou 10 $\mu$l.

En outre chacune des électrodes 4 aura par exemple une surface de l'ordre de quelques dizaines de $\mu m^2$ (par exemple 10 $\mu m^2$) jusqu'à 1 mm$^2$, selon la taille des gouttes à transporter, l'espacement entre électrodes voisines étant par exemple compris entre 1 $\mu$m et 10 $\mu$m.

La structuration des électrodes 4 peut être obtenue par des méthodes classiques des micro-technologies, par exemple par photolithographie.

Des procédés de réalisation de puces incorporant un dispositif selon l'invention peuvent être directement dérivés des procédés décrits dans le document FR - 2 841 063.

Des conducteurs, et notamment des conducteurs 110 peuvent être réalisés par dépôt d'une couche conductrice et gravure de cette couche suivant le motif approprié de conducteurs, avant dépôt de la couche hydrophobe 108.

Les électrodes peuvent être réalisées par dépôts d'une couche métallique (par exemple en un métal choisi parmi Au, Al, ITO, Pt, Cr, Ti, Cu) par photolithographie. Le substrat est ensuite recouvert d'une couche diélectrique, par exemple en $Si_3N_4$ ou en $SiO_2$. Enfin un dépôt d'une couche hydrophobe est effectué, comme par exemple un dépôt de téflon réalisé à la tournette.

Un tel dispositif de déplacement de gouttes peut mettre en oeuvre un réseau bidimensionnel d'électrodes qui vont permettre, de proche en proche, de déplacer des liquides dans ou sur un plan, de les mélanger, afin de réaliser des protocoles complexes.

Dans le cas du mode de réalisation avec caténaires 10 (figures 1A - 3B), un ensemble bidimensionnel (2D) de ces caténaires peut être réalisé au-dessus de l'ensemble 2D d'électrodes 4. Dans le cas du mode de réalisation avec contre-électrode 112 incorporée dans le capot 100 (figures 4 - 5B), cette contre - électrode peut aussi avoir une structure bidimensionnelle.

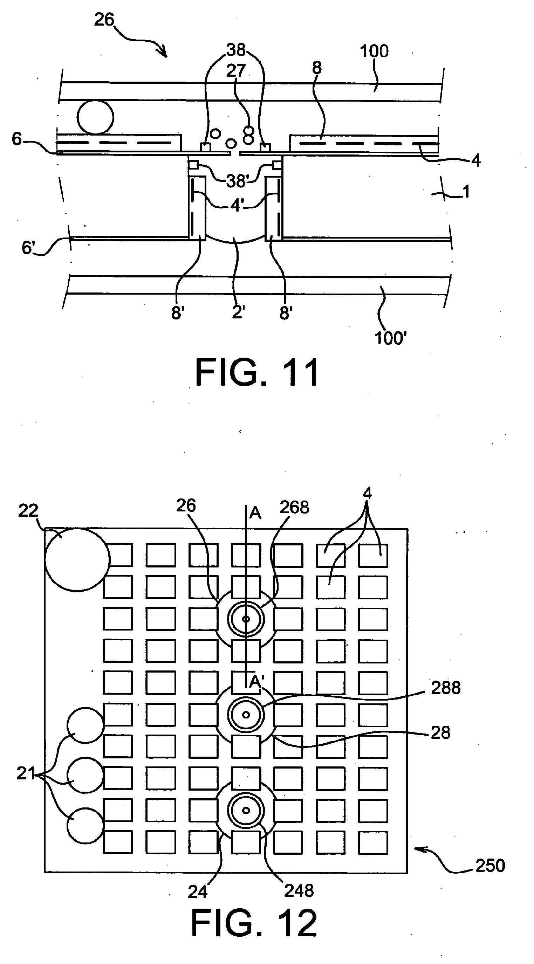

Un exemple d'un tel réseau bidimensionnel sera décrit ci-dessous en liaison avec la figure 12.

Un dispositif de pompage selon l'invention comporte un dispositif d'électromouillage, comme illustré sur les figures 6A (vue de dessus) et 6B (vue de côté).

Un tel dispositif comporte une structure telle que l'une de celles décrites ci-dessus en liaison avec les figures 1A - 5B. La structure illustrée sur les figures 6A et 6B est du type à configuration fermée, avec un substrat 1, dans lequel des électrodes d'électromouillage 4 sont disposées, et un capot 100, qui n'est pas représenté en détail mais qui est similaire, par exemple, au capot de la figure 4 (avec surface hydrophobe 108 et électrode enterrée 112).

Dans ce mode de réalisation, comme dans les suivants, le substrat 1 pourrait également comporter deux substrats accolés dos à dos. Chacun d'entre eux peut en outre avoir une structure selon, par exemple, l'une des figures 1A - 5B, et comporter des électrodes d'électromouillage.

Les électrodes 4 vont permettre, avec la contre électrode du substrat 100, de commander le déplacement d'une goutte 2 localisée entre les deux substrats 1, 100.

Une deuxième goutte 2' peut être disposée par capillarité contre la face 11' du substrat 1 opposée à la face 11 avec laquelle la goutte 2 est en contact.

Un orifice 30 met les deux côtés du substrat 1 en communication.

Le principe du pompage selon l'invention est alors le suivant.

La modification des potentiels des électrodes 4, dans le voisinage d'une goutte 2 va modifier les pressions électrostatiques dans cette goutte; si l'on diminue l'angle de mouillage de cette goutte, le rayon de courbure de son interface avec son environnement va augmenter, entraînant une diminution de la pression en son sein.

Si l'on diminue le potentiel électrique des électrodes 4, la goutte 2 se rétracte, et la pression en son sein va augmenter, du fait de la diminution du rayon de courbure.

Les variations de pression dans la goutte 2 vont se répercuter dans la goutte 2', dont le liquide va ainsi pouvoir être pompé ou refoulé à travers l'orifice 30.

Une variation de pression entre les deux volumes de liquide 2, 2', situés en contact par l'orifice 30 du substrat peut donc être obtenue par électromouillage.

Selon une variante illustrée en figure 7A, des électrodes 4, 4' d'électromouillage sont présentes des deux côtés du substrat 1, permettant de contrôler tant la pression dans la goutte 2 que celle dans la goutte 2'.

Ainsi, les deux gouttes 2, 2' vont pouvoir être déplacées et/ou déformées, de part et d'autre du substrat 1.

Les deux séries d'électrodes peuvent être réalisés avec l'un des procédés déjà décrits ci-dessus. Elles peuvent être pilotées par des moyens de commande tels que déjà décrits ci-dessus.

Le même mécanisme que décrit ci-dessus s'applique alors à la goutte 2' et aux électrodes 4' : si l'on diminue l'angle de mouillage de cette goutte, le rayon de courbure de son interface avec son environnement va augmenter, entraînant une diminution de la pression en son sein ; si l'on diminue le potentiel électrique des électrodes 4', la goutte 2' se rétracte, et la pression en son sein va augmenter, du fait de la diminution du rayon de courbure.

La configuration représentée est fermée sur un des côtés et ouverte en bas (un fil ou caténaire comme sur les figures 1A - 1C peut être utilisé pour la partie inférieure, non confinée, des figures 6B., 7A). Mais il est possible de réaliser une configuration doublement fermée ou confinée ou mixte, avec deux capots 100, 100' de part et d'autre du substrat 1. Il serait également possible d'avoir une configuration ouverte dans la partie supérieure (du côté de la goutte 2) comme dans la partie inférieure, avec d'éventuels caténaires de chaque côté.

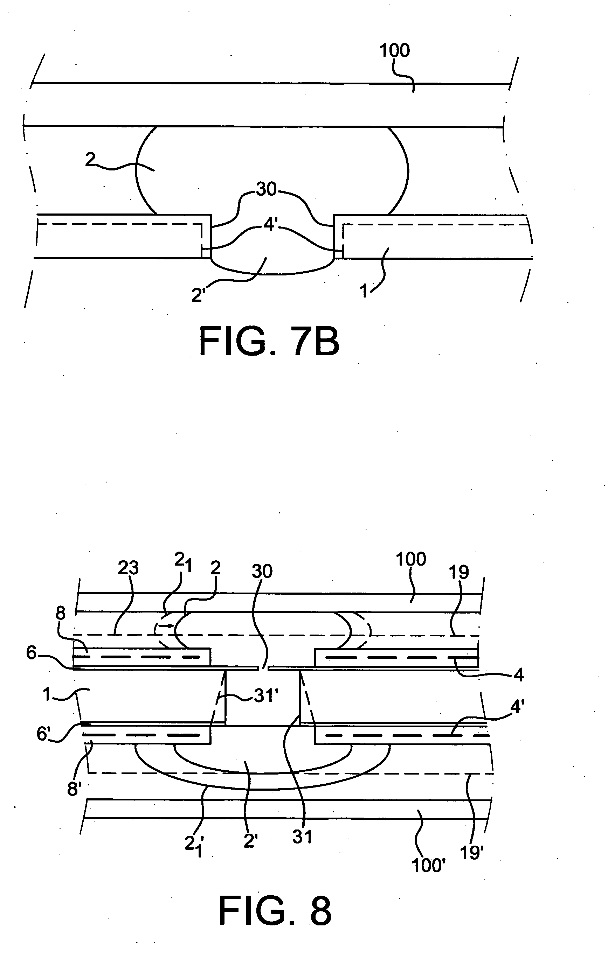

Selon encore une autre variante, illustrée en figure 7B, les électrodes 4' d'électromouillage présentes sur l'une des surfaces du substrat 1, et qui permettent de contrôler la pression dans la goutte 2' sont situées dans la paroi de l'orifice 30. Cette configuration permet d'avoir un petit rayon dans la chambre inférieure en système ouvert.

Le même mécanisme que décrit ci-dessus s'applique alors à la goutte 2' et aux électrodes 4' : si l'on diminue l'angle de mouillage de cette goutte, le rayon de courbure de son interface avec son environnement va augmenter, entraînant une diminution de la pression en son sein ; si l'on diminue le potentiel électrique des électrodes 4', la goutte 2' se rétracte, et la pression en son sein va augmenter, du fait de la diminution du rayon de courbure.

Quel que soit le mode de réalisation envisagé d'un dispositif de pompage selon l'invention, un lien physique existe entre les pressions dans les gouttes 2, 2' et leurs angles de mouillage.

La pression dans la goutte 2 confinée est donnée par la loi de Laplace.

$$P_1 = \gamma \left( \frac{1}{R_1} + \frac{1}{R_2} \right) \qquad (1)$$

où $R_1$ et $R_2$ sont les deux rayons de courbure dans les plans verticaux et horizontaux, ou parallèles et perpendiculaires au plan défini par le substrat 1. Le rayon $R_1$ est directement lié au volume $V_1$ de la goutte 2 par :

$$R_1 = \left( \frac{V_1}{\pi \delta} \right)^{\frac{1}{2}} \qquad (2)$$

où $\delta$ est la distance entre les plaques 1, 100. D'autre part, on peut montrer que :

$$R_2 = \frac{\delta}{-2\cos\theta_0} \qquad (3)$$

où $\theta_0$ est l'angle de contact avec les plaques. La pression dans la goutte est alors:

$$P_1 = \gamma \left( \left( \frac{\pi \delta}{V_1} \right)^{\frac{1}{2}} + \frac{-2\cos\theta_0}{\delta} \right) \qquad (4)$$

Pour augmenter la pression dans la goutte confinée 2, on cherche à rendre $R_2$ petit.

Selon un exemple d'application numérique, en prenant $\theta_0 = 120°$, $\delta = 50$ $\mu$m, $\gamma = 40$ mN/m et $V_1 = 1$ $\mu$l, on trouve une pression $P_1 = 816$ Pa.

La pression dans la goutte 2' est donnée également par la loi de Laplace :

$$P_2 = \frac{2\gamma}{R_3} \qquad (5)$$

En configuration ouverte, la pression dans la goutte 2' est donnée par la loi de Laplace. On a alors :

$$P_1 = \frac{2\gamma}{R_1} \qquad\qquad (5')$$

à l'instar de l'équation (5).

Le rayon de courbure $R_3$ est lié au volume $V_2$ de la goutte et à l'angle de contact $\theta_2$ par:

$$V_2 = \frac{\pi}{3} R_3{}^3 \left(2 - 3\cos\theta_2 + \cos^3\theta_2\right) \qquad (6)$$

La pression dans la goutte 2' est alors:

$$P_2 = \frac{2\gamma}{\left(\dfrac{3V_2}{\pi\left(2 - 3\cos\theta_2 + \cos^3\theta_2\right)}\right)^{\frac{1}{3}}} \qquad (7)$$

Selon un exemple d'application numérique en prenant $\theta_2$=80°, $\gamma$ = 40 mN/m et $V_2$=100µl, on obtient une pression dans la goutte 2' égale à $P_2$ = 20 Pa. La valeur de la tension de surface $\gamma$ = 40 mN/m correspond au cas d'un liquide avec surfactants. Plus généralement cette valeur est comprise entre 10 et 75 mN/m (par exemple : 10 si on est dans l'huile avec des surfactants dans la goutte).

La différence de pression obtenue par le système est donc :

$$\Delta P \cong \gamma \left(\left(\frac{\pi\delta}{V_1}\right)^{\frac{1}{2}} + \frac{-2\cos\theta_0}{\delta}\right) - \frac{2\gamma}{\left(\dfrac{3V_2}{\pi\left(2 - 3\cos\theta_2 + \cos^3\theta_2\right)}\right)^{\frac{1}{3}}} \qquad (8)$$

Avec les valeurs numériques considérées précédemment, on obtient $\Delta P$ = 796 Pa.

Les deux volumes de liquide 2, 2' étant en communication, la différence de pression se rééquilibre ensuite, mais elle est maintenue pendant une durée suffisante pour permettre un effet de pompage.

Un autre mode de réalisation d'un dispositif de pompage selon l'invention est illustré sur la figure 8.

Sur cette figure le substrat 1 est en un matériau semi conducteur tel que le silicium. Sur une de ses faces, un dépôt de diélectrique 6 (par exemple en $Si_3N_4$ ou en $SiO_2$) est percé d'un trou 30 de diamètre par exemple compris entre 1 et 2 µm et, sur la face en vis-à-vis, d'un trou de largeur ou de dimension maximale comprise entre, par exemple, 50 et 1000 µm.

Ce diélectrique est ensuite revêtu d'un réseau d'électrodes 4; ces électrodes sont ensuite passivées, c'est-à-dire isolées électriquement, par exemple par une nouvelle couche de diélectrique sur laquelle on réalise un dépôt hydrophobe 8, par exemple de type téflon.

L'autre face du substrat 1 est traitée de la même manière, avec un isolant 6', des électrodes 4' et une couche hydrophobe 8'.

Une huile 23 permet de prévenir ou de limiter ou d'éviter l'évaporation des gouttes.

Les gouttes 2, dans la partie supérieure, sont, dans le mode de réalisation illustré, confinées, du fait du contact avec les substrats 1, 100, préférentiellement transparents et dont la surface « mouillée » est rendue hydrophobe, par exemple par un dépôt de type téflon. Les substrats 100, 100' sont substantiellement similaires au substrat 100 décrit ci-dessus, par exemple en liaison avec la figure 2.

En fait deux positions de chacune des gouttes sont représentées sur cette figure 8 : une position $2_1$ de la goutte 2 avant compression, et une position $2'_1$ de la goutte 2' après dilatation de cette dernière suite à la compression de la goutte 2.

Ce contact avec un capot 100, 100' à surface hydrophobe et comportant des moyens conducteurs permet de contrôler le potentiel de la goutte 2, 2'.

En variante, ce contrôle peut aussi être fait au moyen d'un caténaire 19 et/ou 19' (représentés en traits interrompus sur la figure 8) traversant la goutte comme indiqué ci-dessus en liaison avec les figures 1A - 1C, donc en configuration ouverte. Cette configuration est également possible pour le pompage avec caténaire. Ce contact ne modifie pas, ou très peu, les pressions dans les gouttes.

Dans l'exemple illustré, l'orifice 30 n'est pas de diamètre ou de dimension maximale, mesuré dans le plan du substrat 1, uniforme suivant la direction perpendiculaire à ce substrat. Une deuxième partie 31 présente un diamètre plus large.

Une application d'un dispositif de pompage selon l'invention à un dispositif de mesure électrique de type « patch - clamp » va maintenant être décrite.

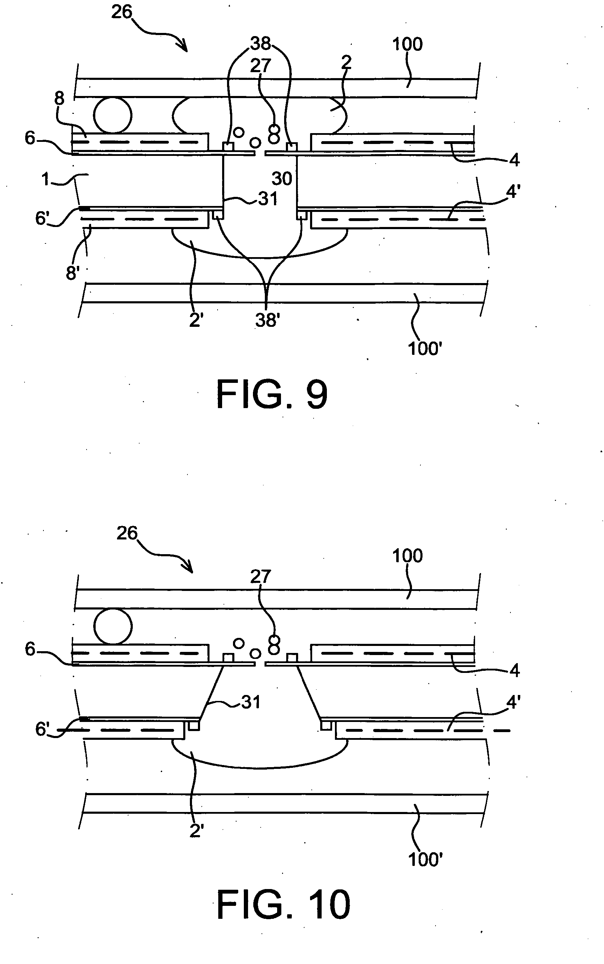

Un tel dispositif est illustré sur la figure 9.

Il comporte une structure de pompage similaire à celle décrite ci-dessus, par exemple en liaison avec la figure 8.

Il comporte en outre des électrodes 38, 38' qui vont permettre de réaliser une mesure de variation de caractéristique électrique du milieu liquide lorsqu'une cellule 27 va être attirée contre le trou 30 et plaquée ou invaginée contre ou dans ce trou (comme la cellule 327' sur la figure 14). En particulier, il est intéressant de réaliser alors une mesure de résistance ou de résistivité du milieu. Typiquement, la présence d'une cellule 27 dans le trou 30 résulte en une variation de résistance de l'ordre de plusieurs MΩ. Si un contact privilégié à l'interface diélectrique / cellule, la résistance atteint des valeurs comprises entre 50 MΩ et 10 GΩ.

Comme déjà indiqué ci-dessus, les électrodes 4 servant à l'électromouillage, ainsi que les électrodes 38 servant à la mesure d'électrophysiologie, sont sur une membrane diélectrique 6 dont le revêtement 8 est hydrophobe et passivé dans les zones de déplacement des gouttes.

Par contre, dans les zones de mesure dans lesquelles sont disposées des électrodes 38, 38', le revêtement 6, 6' est hydrophile et non passivé. Des électrodes en Ag/AgCl conviennent tout à fait pour modifier localement les concentrations d'ions chlore et activer les canaux potassiques de cellules.

Les électrodes de mesure 38, 38' vont permettre d'appliquer une différence de potentiel et de mesurer le courant dans le milieu liquide présent dans les cavités. Des conducteurs, non représentés sur la figure, permettent d'appliquer la tension voulue entre ces deux électrodes. Cette tension est par exemple pilotée ou commandée par les moyens qui permettent de commander ou d'activer les électrodes 4, par exemple un ordinateur de type PC disposant des interfaces appropriées. Ces conducteurs vont permettre également de mesurer la variation de courant entre les électrodes 38, 38' lorsqu'une cellule est amenée sur le site de mesure. Cette variation peut être mémorisée dans des moyens de mémorisation d'un dispositif qui permettra ensuite de traiter et d'analyser les données ainsi prélevées lors des mesures, par exemple en les convertissant en mesure de courant ou de toute autre caractéristique électrique. Un amplificateur de courant peut également être combiné au dispositif, afin de mesurer les variations de courant lors du positionnement de la cellule sur l'orifice 30.

Afin d'isoler électriquement un fragment de membrane d'une cellule, on peut amener, par électromouillage, les cellules 27 - par exemple dans une goutte 2 - vers un site de mesure 26 tel que celui représenté en figure 9. Alors que les cellules sédimentent, une dépression peut être créée de la manière déjà décrite ci-dessus, par le procédé de pompage selon l'invention, entre les chambres inférieure et supérieure ou entre les gouttes 2 et 2'. Une cellule est alors attirée sur le trou 30 de la membrane 6 de diélectrique. Une seule cellule est donc finalement étudiée. Une fois la membrane de la cellule sur le trou 30, celle-ci se déforme, puis s'invagine dans le trou. La résistance électrique mesurée au niveau du contact cellule/diélectrique 6 peut être alors de l'ordre du Giga-Ohm. Cette résistance permet de visualiser, par exemple sur un amplificateur dédié à l'électrophysiologie, des courants de l'ordre du pico-ampère. Ces courants résultent par exemple du passage des ions au travers des protéines canal de la membrane cellulaire.

La réalisation des ouvertures 30, 31 dans le substrat 1 des figures 6A à 10 peut être obtenue par différents modes de gravure.

Si ce substrat est en silicium on utilisera préférentiellement une gravure anisotrope. Cette gravure par KOH ou TMAH a l'avantage d'être réalisée à basse température (<90°C) et ne requiert pas de retraitement des surfaces. En effet, la gravure profonde du silicium met en oeuvre un processus séquentiel qui alterne des étapes de gravure et de passivation par utilisation de polymères fluorés connus pour leur hydrophobie. Les retraitements peuvent être chimiques ou thermiques mais cette dernière alternative est à éviter en vue de l'emploi de métaux. Pour cette raison il est préférable de réaliser une ouverture 31 par gravure humide ou par chimie humide. Ce mode de réalisation est illustré sur la figure 10. Dans ce cas, l'ouverture 31 est en forme de tronc de pyramide. Une telle ouverture peut également être réalisée dans un dispositif de pompage tel que décrit ci-dessus en liaison avec les figures 6A - 8 (voir par exemple l'ouverture 31' représentée en traits interrompus sur la figure 8).

La figure 11 décrit une configuration dans laquelle les électrodes 4', qui permettent la création de la dépression, ne seraient plus situées sur une face du substrat 1 parallèle à un plan principal de ce substrat, mais dans les parois de l'orifice 31. Le principe du pompage reste identique, comme expliqué ci-dessus en liaison avec la figure 7B. Le revêtement 8' est également appliqué aux parois de l'orifice 30, les électrodes 4' étant passivées, tandis que les électrodes 38' restent dépassivées. Une différence de potentiel, permettant la mesure, est appliquée entre les électrodes 38, 38'. Le pompage a lieu comme expliqué ci-dessus en liaison avec les figures 7A et 7B.

Les gouttes 2, 2' peuvent être amenées à un site de mesure 26 par déplacement par électromouillage, à l'aide des électrodes 4, 4', comme expliqué ci-dessus en liaison avec les figures 1A - 1C en configuration ouverte, ou également en configuration fermée (figures 2- 5C). Elles peuvent aussi être amenées ou positionnées manuellement, à l'aide d'une pipette.

Un dispositif selon l'invention peut être incorporé dans un réseau de sites de mesure.

Ainsi, une autre réalisation de l'invention est représentée en vue de dessus en figure 12, sans les capots 100, 100'.

Ce dispositif comporte d'abord un dispositif bidimensionnel de déplacement et de manipulation de gouttes par électromouillage, par exemple du type tel qu'exposé ci-dessus en liaison avec les figures 1A - 5C, et dont seules les électrodes 4 du substrat 1 sont représentées schématiquement.

Les références 22 et 21 désignent plusieurs réservoirs, par exemple un réservoir de cellules 22 et un ou plusieurs réservoir de drogues 21. Un seul réservoir peut dans certains cas être suffisant. Il est également possible de ne pas utiliser de réservoir et d'amener les volumes de liquide à analyser par d'autres moyens, par exemple une pipette.

Le système peut en outre comporter un site 26 de mesure unique, tel que décrit ci-dessus en liaison avec les figures 9 - 11, ou au moins un tel site. Par exemple ce dispositif peut comporter une pluralité de tels sites 24, 26, 28, chacun identique ou similaire à celui décrit ci-dessus en liaison avec les figures 9 - 11. Les références 268, 248, 288 désignent des électrodes de mesure équivalentes à l'électrode 38 des figures 9 - 10.

Les réservoirs 21, 22 sont avantageusement compatibles avec un format de plaques à puits (8, 96, 384, 1586 puits).

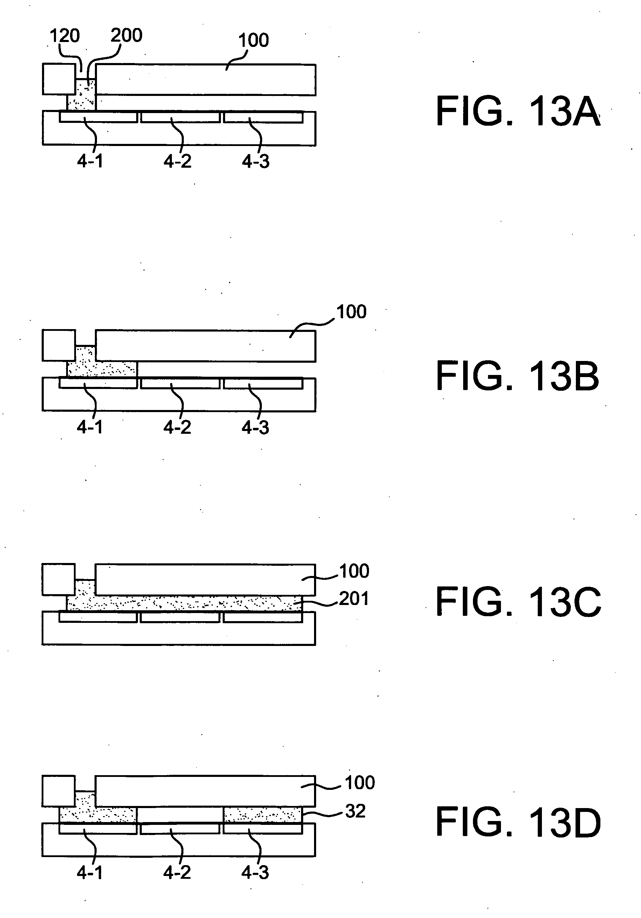

Un exemple de réalisation de ces réservoirs 21 ou 22 va être donné ci-dessous en liaison avec les figures 13A - 13D.

Un liquide 200 à dispenser est déposé dans un puits 120 de ce dispositif (figure 13A). Ce puits est par exemple réalisé dans le capot supérieur 100 du dispositif La partie inférieure, représentée de manière schématique sur les figures 13A - 13D, est par exemple similaire à la structure des figures 1A-1C.

3 électrodes 4-1, 4-2, 4-3, similaires aux électrodes 4 de déplacement de gouttes de liquide, sont représentées sur les figures 11A - 11D.

L'activation de cette série d'électrodes 4-1, 4-2, 4-3 conduit à l'étalement d'une goutte à partir du puits 120, et donc à un segment liquide 201 comme illustré sur la figure 11C.

Puis, on coupe ce segment liquide en désactivant une des électrodes activées (électrode 4-2 sur la figure 11C). On obtient ainsi une goutte 2, comme illustré sur la figure 11D.

On utilise donc une série d'électrodes 4-1, 4-2, 4-3 pour étirer du liquide du réservoir 120 en un doigt 201 (figures 11B et 11C) puis pour couper ce doigt 201 de liquide (figure 11D) et former une goutte 2 qui va pouvoir être emmenée vers tout site de mesure comme décrit ci-dessus.

On peut appliquer ce procédé en insérant des électrodes telles que les électrodes 4 - 1 entre le réservoir 120 et une ou plusieurs électrode 4 - 2 dite électrode de coupure.

Si on n'utilise pas une configuration avec capot supérieur, les microgouttes peuvent être dispensées à l'aide d'une pipette ou d'un automate de dispense.

Un dispositif selon l'invention peut être utilisé pour immobiliser d'autres objets biologiques que des cellules dont on veut étudier l'activité électrophysiologique. Par exemple, l'invention peut s'appliquer à des ovocytes de bovins pour la fécondation in vitro ou encore au déplacement d'embryons. Ces objets biologiques peuvent ensuite être plaqués par aspiration.

Un dispositif selon l'invention permet de déplacer des volumes de fluide sous forme de gouttes. Ces gouttes peuvent intégrer des solides ou des éléments biologiques de grandes tailles tels que des embryons (tailles comprises entre 0.3mm et 1mm). La présente invention permet de déplacer et de bloquer localement un tel objet par les moyens d'aspiration. Ce système peut être utilisé, comme déjà mentionné ci-dessus, pour la manipulation d'embryons ou la fécondation in vitro (FIV) d'ovocytes.

**Revendications**

1.  Procédé d'analyse d'un liquide d'un premier volume de liquide (2) comportant :

    - la mise en contact de ce premier volume de liquide avec une première surface hydrophobe (8) d'un premier substrat (1),
    - le pompage de ce premier volume de liquide (2) à l'aide d'un deuxième volume de liquide, à travers un orifice (30, 31) dudit premier substrat (1), afin de positionner ce premier volume de liquide contre ledit orifice (30, 31), ce premier volume de liquide (2) étant en contact avec ladite première surface hydrophobe dudit premier substrat, une variation de pression entre ledit premier volume de liquide et ledit deuxième volume de liquide (2'), situé en contact avec ledit orifice et une deuxième surface hydrophobe dudit premier substrat, étant obtenue par électromouillage,
- une mesure d'activité électrique dudit premier volume de liquide.

2. Procédé selon la revendication 1, le premier et/ou le deuxième volume de liquide étant confiné, au moins lors du pompage, entre ladite première surface hydrophobe et/ou ladite deuxième surface hydrophobe et un deuxième et/ou un troisième substrat (100, 100').

3. Procédé selon l'une des revendications 1 ou 2, mettant en oeuvre un dispositif d'électromouillage, comportant ledit premier substrat dont la première surface est hydrophobe ou recouverte d'une couche hydrophobe (8), et une pluralité d'électrodes (4) disposées sous ladite couche hydrophobe, le pompage étant obtenu par activation de ces électrodes (4).

4. Procédé selon la revendication 3, la deuxième surface du dispositif d'électromouillage comportant une couche hydrophobe (8'), et une pluralité d'électrodes (4') disposées sous ladite couche hydrophobe.

5. Procédé selon l'une des revendications 1 à 4, les première et deuxième surfaces du substrat étant parallèles entre elles et à un plan défini par le substrat.

6. Procédé selon l'une des revendications 1 à 4, la première surface du substrat étant parallèle à un plan défini par le substrat, et la deuxième surface étant définie par au moins une portion de la paroi dudit orifice (30, 31).

7. Procédé selon l'une des revendications 1 à 6, le premier et/ou le deuxième volume de liquide étant constitué d'une goutte de liquide.

8. Procédé selon la revendication 7, la ou les gouttes étant formés à partir d'un ou de plusieurs réservoirs (21, 22).

9. Procédé selon l'une des revendications 7 ou 8, la ou les gouttes ayant un volume compris entre 1 nl et 100 $\mu$l.

10. Procédé selon l'une des revendications 1 à 9, la mesure d'activité électrique étant réalisée sur une cellule unique (27) contenue dans le premier volume de liquide (2).

11. Procédé selon la revendication 10, la mesure étant une mesure sur un canal ou des canaux de la cellule ou d'une membrane cellulaire.

12. Procédé selon l'une des revendications 1 à 9, la mesure d'activité électrique étant réalisée sur un objet biologique tel qu'un embryon ou un ovocyte de bovin.

13. Dispositif de pompage de volumes de liquide, comportant :

- un substrat (1), présentant une première et une deuxième surface, dont au moins une est hydrophobe, ou recouverte d'une couche hydrophobe, et au moins un orifice (30, 31) traversant ledit substrat,
- des moyens (4) pour déplacer, par électromouillage, au moins un volume de liquide sur une desdites faces du substrat, **caractérisé par**
- des moyens de mesure d'activité électrique d'un volume de liquide, associés à au moins un orifice, afin de réaliser une mesure de variation de caractéristique électrique du milieu liquide lorsque ledit volume de liquide est attiré contre ledit orifice.

14. Dispositif selon la revendication 13, ledit orifice (30, 31) comportant une première partie, ayant une première dimension maximale, et une deuxième partie, ayant une deuxième dimension maximale, supérieure à la première.

15. Dispositif selon la revendication 13 ou 14, les moyens (4) de déplacement de gouttes par électromouillage comportant une pluralité d'électrodes (4) disposées sous ladite surface ou couche hydrophobe.

16. Dispositif selon l'une des revendications 13 à 15, la deuxième surface du dispositif d'électromouillage étant hydrophobe ou comportant une couche hydrophobe (8'), et une pluralité d'électrodes (4') disposées sous ladite surface ou couche hydrophobe.

17. Dispositif selon l'une des revendications 13 à 16, les première et deuxième surfaces du substrat étant parallèles entre elles et à un plan défini par le substrat.

18. Dispositif selon l'une des revendications 13 à 16, la première surface du substrat étant parallèle à un plan défini par le substrat, et la deuxième surface étant définie par au moins une portion de la paroi dudit orifice (30, 31).

19. Dispositif selon l'une des revendications 13 à 18, comportant en outre une première couche diélectrique (6), en partie sous la surface ou la couche hydrophobe (8).

20. Dispositif selon la revendication 19, comportant en outre une deuxième couche diélectrique (6'), disposée sur une surface parallèle à la première surface.

21. Dispositif selon l'une des revendications 13 à 20, comportant en outre un deuxième substrat et/ou un troisième substrat (100, 100'), disposé en regard de la première et/ou de la deuxième surface.

22. Dispositif selon la revendication 21, le deuxième et/ou le troisième substrat comportant en outre une couche hydrophobe superficielle (108, 108').

23. Dispositif selon la revendication 21 ou 22, le deuxième et/ou le troisième substrat comportant en outre une électrode (112, 112').

24. Dispositif d'analyse d'un premier volume de liquide, comportant un ou plusieurs dispositif selon l'une des revendications 13 à 23, et des moyens (38, 38') de mesure d'activité électrique d'un volume de liquide associés à au moins un orifice (30, 31).

25. Dispositif selon la revendication 24, les moyens de mesure d'activité électrique associés à au moins un orifice comportant des première et deuxième électrodes (38, 38').

26. Dispositif selon l'une des revendications 13 à 25, comportant en outre au moins un réservoir (21, 22) de liquide et des moyens pour amener des gouttes de liquide depuis ce réservoir ou depuis au moins un de ces réservoirs vers l'un des orifices (30, 31).

27. Dispositif selon la revendication 26, les moyens pour amener des gouttes de liquide vers l'un des orifices étant des moyens (4) de déplacement de gouttes par électromouillage.


**Claims**

1. A method for analyzing a liquid from a first volume of liquid (2) including:

   - contacting this first volume of liquid with a hydrophobic surface (8),
   - pumping this first volume of liquid by means of a second volume of liquid, through an orifice (30, 31) of said first substrate (1), in order to position it against said orifice (30, 31), said first volume of liquid (2) being in contact with a first hydrophobic surface of said substrate, a pressure variation between said first volume of liquid and a second volume of liquid (2'), located in contact with said orifice and a second hydrophobic surface of said substrate, being achieved by electrowetting,
   - measuring the electric activity of said first volume of liquid.

2. The method according to claims 1, the first and/or the second volume of liquid being confined, at least during pumping, between said first hydrophobic surface and/or said second hydrophobic surface and a second and/or a third substrate (100, 100').

3. The method according to any of claims 1 or 2, making use of an electrowetting device, including said first substrate, the first surface of which is hydrophobic or covered with a hydrophobic layer (8), and a plurality of electrodes (4) positioned under said hydrophobic layer, pumping being obtained by activating these electrodes (4).

4. The method according to claim 3, the second surface of the electrowetting device including a hydrophobic layer (8'), and a plurality of electrodes (4') positioned under said hydrophobic layer.

5. The method according to any of claims 1 to 4, the first and second surfaces of the substrate being parallel to each other and to a plane defined by the substrate.

6. The method according to any of claims 1 to 4, the first surface of the substrate being parallel to a plane defined by the substrate, and the second surface being defined by at least one portion of the wall of said orifice (30, 31).

7. The method according to any of claims 1 to 6, the first and/or the second volume of liquid consisting of a drop of liquid.

8. The method according to claim 7, the drop(s) being formed from one or more reservoirs (21, 22).

9. The method according to any of claims 7 or 8, the drop (s) having a volume between 1 nl and 100 μl.

10. The method according to any of claims 1 to 9, the electric activity measurement being performed on a single cell (27) contained in the first volume of liquid (2).

11. The method according to claim 10, the measurement being a measurement on one channel or on channels of the cell or a cell membrane.

12. The method according to any of claims 1 to 11, the electric activity measurement being performed on a biological object such as an embryo or a bovine ovocyte.

13. A device for pumping volume of liquid, including:

   - a substrate (1), having a first and a second surface, at least one of which is hydrophobic or covered with a hydrophobic layer, and at least one orifice (30, 31) crossing through said substrate,
   - means (4) for displacing, by electrowetting, at least one volume of liquid on one of said faces of the substrate,

   **Characterized by**:

   - means for measuring the electrical activity of a volume of liquid associated with at least one orifice, to achieve measurement of the variation of an electric characteristic of the liquid medium when said volume of liquid is attracted against said hole.

14. The device according to claim 13, said orifice (30, 31) including a first portion, with a first maximum dimension, and a second portion, having a second maximum dimension, larger than the first.

15. The device according to claim 13 or 14, the means (4) for displacing drops by electrowetting including a plurality of electrodes (4) positioned under said hydrophobic layer or surface.

16. The device according to any of claims 13 to 15, the second surface of the electrowetting device being hydrophobic or including a hydrophobic layer (8'), and a plurality of electrodes (4') positioned under said hydrophobic layer or surface.

17. The device according to any of claims 13 to 16, the first and second surfaces of the substrate being parallel to each other and in a plane defined by the substrate.

18. The device according to any of claims 13 to 16, the first surface of the substrate being parallel to a plane defined by the substrate, and the second surface being defined by at least one portion of the wall of said orifice (30, 31).

19. The device according to any of claims 13 to 18, further including a first dielectric layer (6), in part under the hydrophobic layer (8) or surface

20. The device according to claim 19, further including a second dielectric layer (6'), positioned on a surface parallel to the first surface.

21. The device according to any of claims 13 to 20, further including a second substrate and/or a third substrate (100, 100'), positioned facing the first and/or the second surfaces.

22. The device according to claim 21, the second and/or the third substrate further including a hydrophobic surface layer (108, 108').

23. The device according to claim 21 or 22, the second and/or the third substrate further including an electrode (112, 112').

24. A device for analyzing a first volume of liquid, including one or more devices according to any of claims 13 to 23, and means (38, 38') for measuring the electrical activity of a volume of liquid associated with at least one orifice (30, 31).

25. The device according to claim 24, the electric activity measurement means associated with at least one orifice including first and second electrodes (38, 38').

26. The device according to any of claims 13 to 25, further comprising at least one reservoir (21, 22) of liquid and means for bringing drops of liquid from this reservoir or from at least one of these reservoirs towards one of the orifices (30, 31).

27. The device according to claim 26, the means for bringing drops of liquid towards one of the orifices being means (4) for displacing drops by electrowetting.


**Patentansprüche**

1. Verfahren zur Analyse einer Flüssigkeit mit einem ersten Flüssigkeitsvolumen (2), umfassend:

   - das In-Kontakt-Bringen dieses ersten Flüssigkeitsvolumens mit einer ersten hydrophoben Oberfläche (8) eines ersten Substrats (1),
   - das Pumpen dieses ersten Flüssigkeitsvolumens (2) mit Hilfe eines zweiten Flüssigkeitsvolumens durch eine Öffnung (30, 31) des ersten Substrats (1) hindurch, um dieses erste Flüssigkeitsvolumen gegen die Öffnung (30, 31) zu positionieren, wobei dieses erste Flüssigkeitsvolumen (2) im Kontakt ist mit der ersten hydrophoben Oberfläche des ersten Substrats, wobei eine Variation des Drucks zwischen dem ersten Flüssigkeitsvolumen und dem zweiten Flüssigkeitsvolumen (2'), das sich im Kontakt mit der Öffnung und einer zweiten hydrophoben Oberfläche des ersten Substrats befindet, mittels Elektrobenetzung erhalten wird,
   - eine Messung der elektrischen Aktivität des ersten Flüssigkeitsvolumens.

2. Verfahren nach Anspruch 1, wobei das erste und/oder das zweite Flüssigkeitsvolumen wenigstens während des Pumpens begrenzt ist/sind zwischen der ersten hydrophoben Oberfläche und/oder der zweiten hydrophoben Oberfläche und einem zweiten und/oder einem dritten Substrat (100, 100').

3. Verfahren nach einem der Ansprüche 1 oder 2, das eine Elektrobenetzungsvorrichtung verwendet, umfassend das erste Substrat, dessen erste Oberfläche hydrophob oder mit einer hydrophoben Schicht (8) bedeckt ist, und eine Mehrzahl von Elektroden (4), die unter der hydrophoben Schicht angeordnet sind, wobei das Pumpen durch Aktivierung dieser Elektroden (4) erzielt wird.

4. Verfahren nach Anspruch 3, wobei die zweite Oberfläche der Elektrobenetzungsvorrichtung eine hydrophobe Schicht (8') sowie eine Mehrzahl von Elektroden (4') umfasst, die unter der hydrophoben Schicht angeordnet sind.

5. Verfahren nach einem der Ansprüche 1 bis 4, wobei die erste und die zweite Oberfläche des Substrats parallel zueinander und zu einer durch das Substrat definierten Ebene sind.

6. Verfahren nach einem der Ansprüche 1 bis 4, wobei die erste Oberfläche des Substrats parallel ist zu einer durch das Substrat definierten Ebene, und wobei die zweite Oberfläche durch wenigstens einen Bereich der Wand der Öffnung (30, 31) definiert ist.

7. Verfahren nach einem der Ansprüche 1 bis 6, wobei das erste und/oder das zweite Flüssigkeitsvolumen durch einen Flüssigkeitstropfen gebildet ist/sind.

8. Verfahren nach Anspruch 7, wobei der oder die Tropfen ausgehend von einem oder mehreren Reservoirs (21, 22) gebildet ist/sind.

9. Verfahren nach einem der Ansprüche 7 oder 8, wobei der oder die Tropfen ein Volumen hat/haben, das zwischen 1 nl und 100 µl entfalten ist.

10. Verfahren nach einem der Ansprüche 1 bis 9, wobei die Messung der elektrischen Aktivität bei einer einzigen Zelle (27) durchgeführt wird, die in dem ersten Flüssigkeifisvolumen (2) enthalten ist.

11. Verfahren nach Anspruch 10, wobei die Messung eine Messung bei einem Kanal oder bei Kanälen der Zelle oder einer Zellmembran ist.

12. Verfahren nach einem der Ansprüche 1 bis 9, wobei die Messung der elektrischen Aktivität bei einem biologischen Objekt wie z. B. einem Rinderembryo oder einem Rinderoozyt durchgeführt wird.

13. Vorrichtung zum Pumpen von Flüssigkeitsvolumina, umfassend:

- Ein Substrat (1), das eine erste und eine zweite Oberfläche aufweist, von denen wenigstens eine hydrophob oder mit einer hydrophoben Schicht bedeckt ist, sowie wenigstens eine Öffnung (30, 31), die das Substrat durchsetzt,
- Mittel (4) zur Verlagerung wenigstens eines Flüssigkeitsvolumens auf einer der Flächen des Substrats mittels Eiektrabenetzung, **gekennzeichnet durch**
- Mittel zum Messen der elektrischen Aktivität eines Flüssigkeitsvolumens, die wenigstens einer Öffnung zugeordnet sind, um eine Messung der Variation der elektrischen Eigenschaft der flüssigen Umgebung durchzuführen, wenn das Flüssigkeitsvolumen gegen die Öffnung angezogen wird.

14. Vorrichtung nach Anspruch 13, wobei die Öffnung (30, 31) einen ersten Teil mit einer ersten maximalen Abmessung sowie einen zweiten Teil mit einer zweiten maximalen Abmessung größer als die erste umfasst.

15. Vorrichtung nach Anspruch 13 oder 14, wobei die Mittel (4) zur Verlagerung von Tropfen mittels Elektrobenetzung eine Mehrzahl von Elektroden (4) umfassen, die unter der Oberfläche oder hydrophoben Schicht angeordnet sind.

16. Vorrichtung nach einem der Ansprüche 13 bis 15, wobei die zweite Oberfläche der Elektrobenetzungsvorrichtung hydrophob ist oder eine hydrophobe Schicht (8') umfasst, und eine Mehrzahl von Elektroden (4') unter der Oberfläche oder hydrophoben Schicht angeordnet ist.

17. Vorrichtung nach einem der Ansprüche 13 bis 16, wobei die erste und die zweite Oberfläche des Substrats zueinander und zu einer durch das Substrat definierten Ebene parallel sind.

18. Vorrichtung nach einem der Ansprüche 13 bis 16, wobei die erste Oberfläche des Substrats parallel zu einer durch das Substrat definierten Ebene ist, und wobei die zweite Oberfläche durch wenigstens einen Bereich der Wand der Öffnung (30, 31) definiert ist.

19. Vorrichtung nach einem der Ansprüche 13 bis 18, die ferner teilweise unter der Oberfläche oder der hydrophoben Schicht (8) eine erste dielektrische Schicht (6) umfasst.

20. Vorrichtung nach Anspruch 19, ferner umfassend eine zweite dielektrische Schicht (6'), die auf einer zur ersten Oberfläche parallelen Oberfläche angeordnet ist.

21. Vorrichtung nach einem der Ansprüche 13 bis 20, ferner umfassend ein zweites Substrat und/oder ein drittes Substrat (100, 100'), das gegenüber der ersten und/oder der zweiten Oberfläche angeordnet ist/sind.

22. Vorrichtung nach Anspruch 21, wobei das zweite und/oder das dritte Substrat ferner eine hydrophobe Oberflächenschicht (108, 108') umfasst/umfassen.

23. Vorrichtung nach Anspruch 21 oder 22, wobei das zweite und/oder das dritte Substrat ferner eine Elektrode (112, 112') umfasst/umfassen.

**24.** Vorrichtung zur Analyse eines ersten Flüssigkeitsvolumens, umfassend eine oder mehrere Vorrichtungen nach einem der Ansprüche 13 bis 23 sowie Mittel (38, 38') zum Messen der elektrischen Aktivität eines Flüssigkeitsvolumens, die wenigstens einer Öffnung (30, 31) zugeordnet sind.

**25.** Vorrichtung nach Anspruch 24, wobei die Mittel zur Messung der elektrischen Eigenschaft, die wenigstens einer Öffnung zugeordnet sind, eine erste und eine zweite Elektrode (38, 38') umfassen.

**26.** Vorrichtung nach einem der Ansprüche 13 bis 25, ferner umfassend wenigstens ein Flüssigkeitsreservoir (21, 22) sowie Mittel zum Bringen von Flüssigkeitstropfen aus diesem Reservoir oder aus wenigstens einem dieser Reservoirs zu einer der Öffnungen (30, 31) hin.

**27.** Vorrichtung nach Anspruch 26, wobei die Mittel zum Bringen von Flüssigkeitstropfen zu einer der Öffnungen hin Mittel (4) zur Verlagerung von Tropfen mittels Elektrobenetzung sind.

## FIG. 1A

## FIG. 1B

## FIG. 1C

FIG. 2

FIG. 3A

FIG. 3B

FIG. 4

FIG. 5A

FIG. 5B

FIG. 6A

FIG. 6B

FIG. 7A

FIG. 7B

FIG. 8

## FIG. 9

## FIG. 10

# FIG. 11

# FIG. 12

120    200                100

**FIG. 13A**

4-1        4-2        4-3

100

**FIG. 13B**

4-1        4-2        4-3

100
201

**FIG. 13C**

100
32

**FIG. 13D**

4-1        4-2        4-3

FIG. 14

EP 1 891 329 B1

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 04038409 A
- WO 2004038409 A2
- WO 2004029608 A
- FR 2841063

**Littérature non-brevet citée dans la description**

- **M.G. Pollack ; A.D. Shendorov ; R.B. Fair.** Electro-wetting-based actuation of droplets for integrated microfluidics. *Lab on Chip,* 2002, vol. 2 (1), 96-101